# EUROPEAN PATENT APPLICATION

(11) **EP 3 330 268 A1**
(43) Date of publication of application: **06.06.2018**
(21) Application number: 16820892.4
(22) Date of filing: 06.07.2016
(51) Int. Cl.: C07D 471/04, C09B 7/02, G01N 21/64, C09K 11/06, F21V 9/16

(54) **ORGANOGEL BASED ON MOLECULES DERIVED FROM 7,7'-DIAZAISOINDIGO**

(30) Priority: 06.07.2015 ES 201530966
(71) Applicant: Consejo Superior De Investigaciones Científicas (CSIC), 28006 Madrid (ES)
(72) Inventor: GARCIA FRUTOS, Eva María, 28049 Madrid (ES)
(74) Representative: Pons
(86) International application number: PCT/ES2016/070504
(87) International publication number: WO 2017/005956

(57) **Abstract**

The invention relates to: a compound derived from 7,7'-diazaisoindigo; an organogel, formed by these compounds, having an aggregation-induced emission (AIE) in the red region of the visible spectrum (600-800nm); and a xerogel produced by drying said organogel. Due to these properties, said organogel or xerogel can be applied to optoelectronic devices or fluorescent sensors.

## Description

The present invention refers to a compound derived from 7,7'-diazaisoindigo soluble in polar organic solvents which, upon generating an organogel, has an aggregation-induced emission (AIE) in the red region of the visible spectrum (600-800 nm). Due to these properties, said organogel can be applied to optoelectronic devices or to fluorescent sensors.

### STATE OF THE ART

In recent years there has been a huge surge of interest in the self-assembly of low-molecular-weight organogels (LMOG). Their unique supramolecular organisation allows a great variety of potential applications. An enormous amount of different organogels having different functional groups such as amides, hydroxyls, ureas, carboxylic acids, peptides, sugars, cholesterol, large chiral/achiral aliphatic chains (Chem. Rev. 2014, 114, 1973-2129), etc., has been described. These groups help to form organogels through the generation of non-covalent interactions, such as π-π stackings, hydrogen bonds, hydrophobic interactions, Van der Waals interactions, etc.

Moreover, major scientific efforts have been made in the development of a variety of π-conjugated systems aimed at the preparation of organogels, since these aromatic moieties allow modulation of their physical properties. Descriptions have been made of organogels with charge transfer-induced mobility, electric conductivity and luminescence properties for different applications such as, as optoelectronic devices, fluorescence sensors, formation of cellular images and logical states,inter alia. Among all these applications, those focused on photonics have been the most extensively studied, due to the fact that the organogelation process entails significant changes in fluorescent emission. Most of these aggregate compounds have a common characteristic known as reduced emission due to "aggregation-caused quenching" (ACQ), said effect being destructive to their practical applications. The opposite effect has also been observed, known as "aggregation-induced emission" (AIE) or "aggregation-induced enhanced emission" (AIEE). In this case, promising luminescent materials are not (or scarcely) emitters in very diluted solutions, but become highly efficient emitters in concentrated solutions. The reasons for this phenomenon are restrictions in intramolecular rotations (IMR), the formation of aggregates, strengthened planarity, the suppression of twisted intramolecular charge-transfer (TICT) or the existence of excited-state intramolecular proton-transfer (ESIPT).

A huge variety of molecules with AIE have been developed, having great structural diversity: classic luminogens with AIE such as xylols, tetraphenylethylene (TPE) and cyanostilbene derivatives or non-classic luminogens such as 1,3,4-oxadiazole derivatives, carbazoles and dendritic systems (Zhao, Z.; Lam, J. W. Y.; Tang, B. Z. Soft Mater. 2013, 9, 4564).

The document ES201530187 discloses a series of 7-azaindole derivatives with self-assembly capacity for giving rise to organogels and xerogels with important fluorescent properties.

### DESCRIPTION OF THE INVENTION

In a first aspect, the present invention refers to a compound of formula (I): 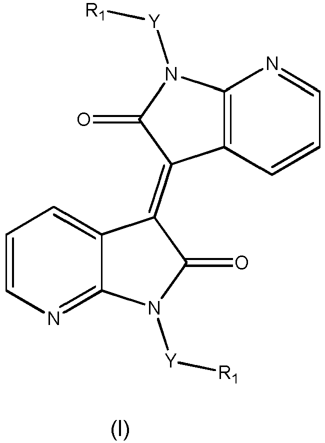 where Y is selected from CH₂, O, NH, C(O), S, S(O), NHC(O), (O)CNH and R₁ is a C₁-C₁₂ alkyl.

The term "alkyl" refers, in the present invention, to aliphatic, linear or branched chains having 1 to 12 carbon atoms, for example, methyl, ethyl, *n*-propyl, *i*-propyl, *n*-butyl, *terc-*butyl, sec-butyl, pentyl, dodecyl, etc. The alkyl group preferably has 3 to 11 carbon atoms. The alkyl groups may be optionally substituted by one or more substituents such as halogen, hydroxyl, azide, carboxylic acid or a substituted or non-substituted group, selected from amine, amide, carboxylic ester, thiol, acylamine and carboxamide.

In a preferred embodiment, Y is CH₂.

In a more preferred embodiment, R₁ is a C₃-C₁₁ alkyl and even more preferably heptyl.

In another preferred embodiment, the compound of formula (I) has the following formula:

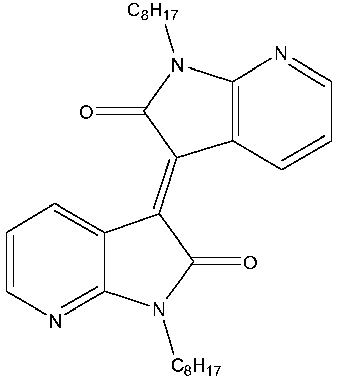

Another aspect of the invention refers to the use of a compound of formula (I) for manufacturing fluorescent materials.

Another aspect of the invention refers to an organogel comprising at least one compound of formula (I), as described previously.

Another aspect of the invention refers to a xerogel characterised in that it is the previously described organogel, dessicated.

In the present invention, gels are understood to be viscoelastic structures formed from a three-dimensional interconnected network and a solvent, which is the majority component; the solid appearance of the gel is the result of the occlusion and adhesion of the liquid to the surface of the solid three-dimensional matrix; the formation of this matrix is the result of the cross-linking of polymer fibres formed from the bonding of the molecules by means of physical or chemical interactions. They are understood as organogels if the solvent used is organic in nature. Xerogel is understood as a solid formed from a gel which has been subjected to a drying process.

The compound of formula (I) is soluble in polar organic solvents such as chloroform or dichloromethane, giving rise to homogeneous solutions at room temperature. However, it precipitates in apolar solvents such as cyclohexane at room temperature. Heating these solutions in apolar solvents and subsequently cooling them induces the formation of gel-type materials; during the sol-gel phase an AIE phenomenon is induced that makes the organogel emit fluorescence in the red spectrum. The batochromic shift and emission in the red spectrum of the organogel of the invention is presumably due to the self-association of 7,7'-diazaisoindigo.

In the solid state, the xerogel formed from the organogel of the compound of formula (I) exhibits an emission in the red spectrum, due to which it could be used as a red solid-state emitter. Therefore, the gel of the present invention is useful for obtaining an emitter material for different applications such as optoelectronic devices, fluorescent sensors, bioimaging, organic light-emitting diode, etc.

Another aspect of the invention refers to a material comprising the gel formed from a compound of formula (I) as described previously.

Another aspect of the invention refers to a device comprising the previously described material.

Another aspect of the invention refers to the use of the material comprising the gel formed from a compound of formula (I) for manufacturing optoelectronic devices, fluorescent sensors, etc.

Throughout the description and claims, the word "comprises" and its variants are not intended to exclude other technical characteristics, additives, components or steps. For the persons skilled in the art, other objects, advantages and characteristics of the invention will be inferred partly from the description and partly from the practice of the invention. The following examples and figures are provided by way of illustration and are not intended to be limiting of the present invention.

### BRIEF DESCRIPTION OF THE FIGURES

**FIG. 1****.** a) Image of the organogel obtained from compound **1** (at 2.75% w/w) in cyclohexane at room temperature and following the "inverted tube" procedure b) SEM images of a dried gel of **1** in cyclohexane.
**FIG. 2****.** Absorption **1** in cyclohexane 2x10⁻⁵ M.
**FIG. 3****.** Emission spectrum of 1 (λ_{exc} = 328 nm) in cyclohexane, the continuous line is (2x10⁻⁴ M), the dashed line is the gel at 4.6x10⁻² M in cyclohexane.
**FIG. 4****.** Emission spectrum of the gel state and sol state of **1** at different temperatures in cyclohexane.
**FIG. 5****.** Optical microphotograph of the crystals obtained upon cooling hot acetone.
**FIG. 6****.** Packed crystallographic structure of compound **1**

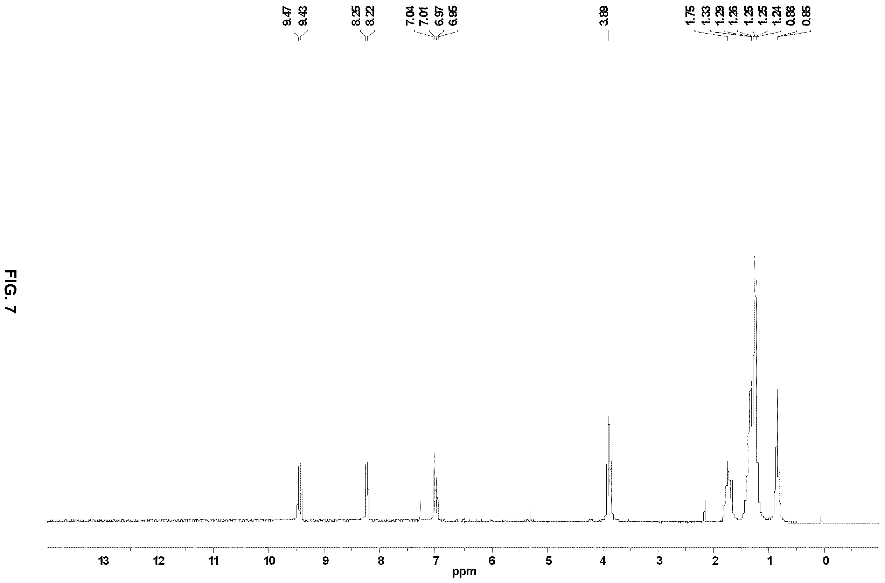

**FIG. 7**. ¹H NMR spectrum of **1** in CDCl₃.

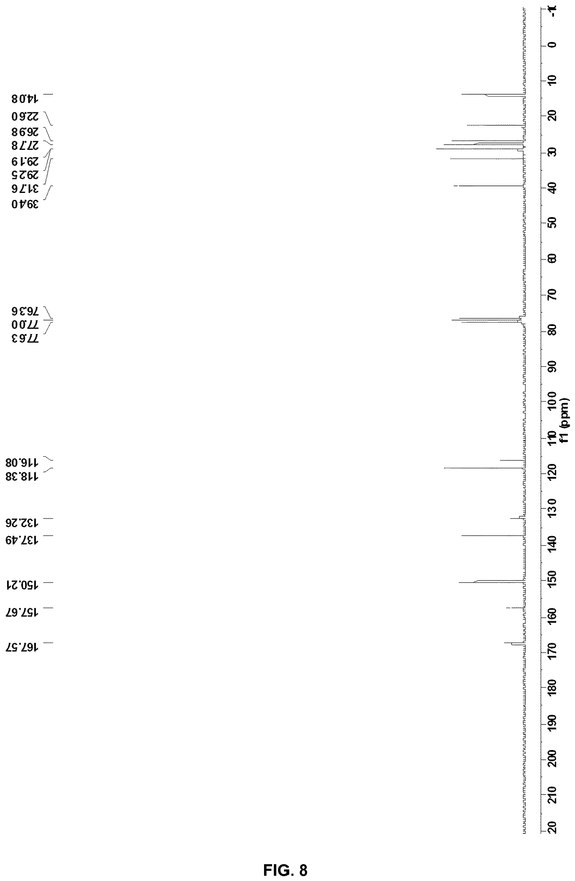

**FIG. 8****.** ¹³C NMR spectrum of **1** in CDCl₃.

### EXAMPLES

The invention is illustrated hereunder by means of assays that demonstrate the effectiveness of the product of the invention.

### Example 1: synthesis of compound (1)

The synthesis of *N*-dioctyl 7,7'-diazaisoindigo (**1**, Scheme 1) was carried out by means of the alkylation of 7,7'-diazaisoindigo by means of 1-iodooctane in the presence of K₂CO₃ and DMF at 100°C for 16 hours.

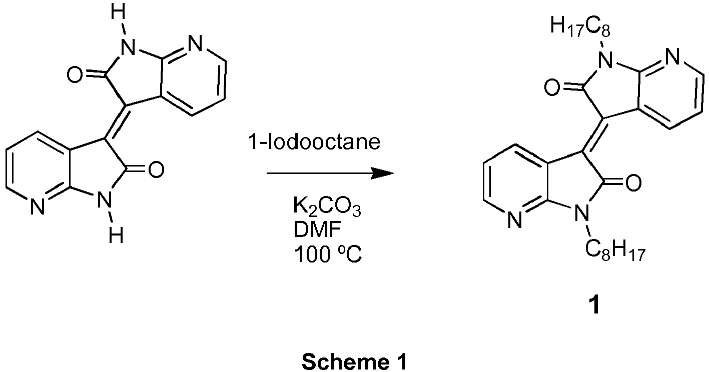

A mixture of 7,7'-diazaisoindigo (25 mg, 0.09 mmol), 1-iodooctane (0.04 ml, 0.21 mmol) and K₂CO₃ (39.2 mg, 0.28 mmol) in 2 ml of DMF was heated to 100°C for 16 hours. The red solution was dissolved in CH₂Cl₂, washed with water and dried with anhydrous MgSO₄. The solvent was evaporated and the residue was chromatografied on the silica gel (hexane:acetone, 5:1) to give a red solid (1) (30 mg, 65%):
**(*E*)-1,1'-dioctyl-[3,3'-bipyrrolo[2,3-b]pyridinylidene]-2,2'(1*H*.1'*H*)-dione** (**1)**: ¹H NMR (200 MHz, CDCl₃,) *δ* 9.46 (d, *J* = 8Hz, 2H), 8.24 (d, *J* = 8Hz, 2H), 7.02 (dd, *J* = 8Hz, 2H), 3.90 (t, *J* = 7.5Hz, 4H), 1.76 (m, 4H), 1.24 (m, 20H), 0.87 (t, *J* = 6.5Hz, 6H); ¹³C NMR (50 MHz, CDCl₃) *δ* 167.6, 157.7, 150.2, 137.5, 132.3, 118.4, 116.1, 39.4, 31.8, 29.2, 29.2, 27.8, 27.0, 22.6, 14.1; UV-vis (CH₂Cl₂, 25°C) λₘₐₓ (ε) 283 (30690), 327 (12034), 477 (5069); MALDI-TOF MS m/z 489 (M⁺); HRMS (MALDI-TOF) calculated for C₃₀H₄₀N₄O₂: 489.3224, found: 489.3240.

### Example 2: synthesis and study of the organogel properties based on compound (1)

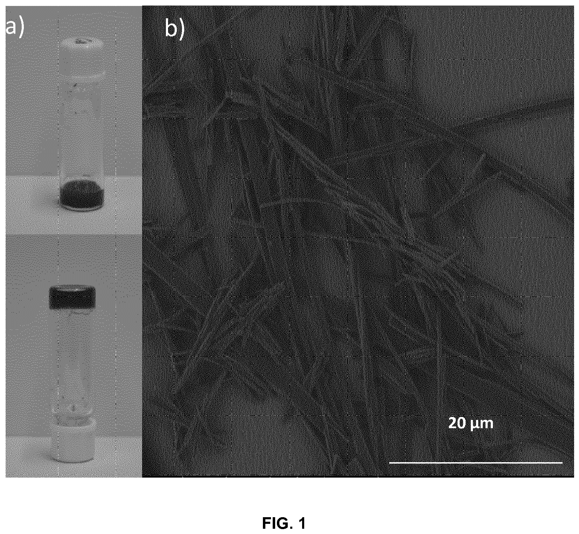

In order to obtain the organogel based on compound (**1**), the powder of this compound was dissolved (at 2.75% w/w) in cyclohexane, used as an apolar solvent, by heating, forming non-fluid gel-type materials after cooling. Additionally, this organogel of **1** is opaque and red in colour, wherein the sol-gel interconversion cycle was assayed by means of the "inverted tube" procedure (Figure 1a).

In order to obtain a visual understanding of the aggregation, the morphology of the dried gel (xerogel) was microscopically examined by means of field emission scanning electron microscopy (FE-SEM) (Figure 1b). The xerogel of **1,** prepared by means of slow cyclohexane evaporation in the gel state, was not homogeneous and long fibres were easily found inside the films (Figure 1b). In the SEM analysis, the gel was transferred to a silicon substrate and the solvent was slowly evaporated again to give a xerogel.

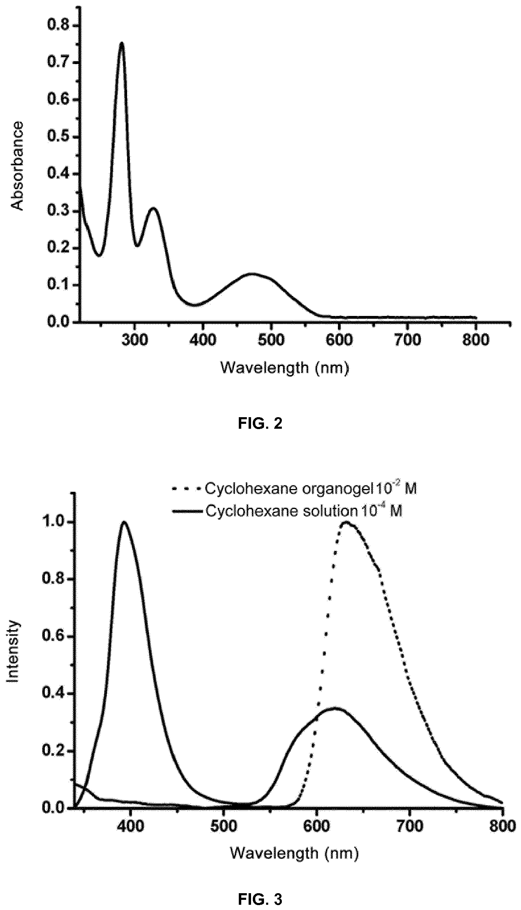

The spectroscopic characterisations of **1** both in solution and in solid state were studied. The electronic absorption spectrum of **1** in cyclohexane showed three absorption bands in 280, 327, 470 nm (10⁻⁵ M) (Figure 2). The absorption band of the xerogel **1** in film on quartz slides showed three absorption bands at 287, 329 and 507 nm, with a broader band absorption spectrum in comparison to the solution state. The absorption spectrum of the xerogel in film state is more batochromically shifted compared to that in a solution state, presumably due to the increased intermolecular interactions between neighbour molecules in the solid state.

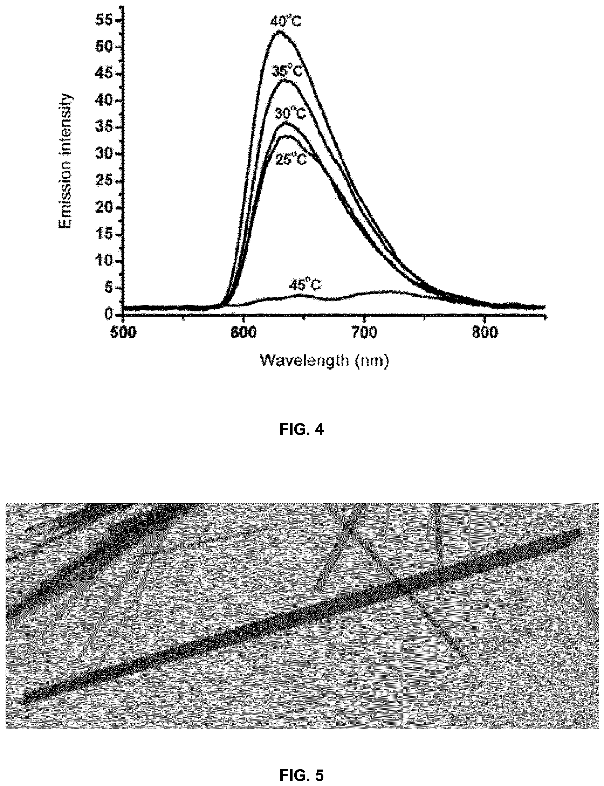

The fluorescence spectrum of **1** in a cyclohexane solution (2x10⁻⁴ M) showed two bands, with a maximum at 392 and 618 nm (λ_{exc} = 328 nm). However, compound **1** at 4.6x10⁻² M in cyclohexane forms an organogel, which emits in the red region of the spectrum with a batochromic shift with respect to the diluted solution, which shows a typical AIE characteristic. (Figure 3). However, when this organogel is heated in cyclohexane, it can be observed how its emission in gel state is eliminated, observing that it is scarcely fluorescent in the sol state (Figure 4). The gel-sol state transition is between 40-45°C.

Therefore, it is conjectured that the supramolecular organogel gelation and formation process induced an aggregation-induced emission phenomenon (AIE). Said AIE behaviour was found during sol-gel phase transition.

Moreover, the emission spectrum of the xerogel of **1,** obtained from the gel state in cyclohexane, also exhibits a considerable batochromic shift compared to the solution diluted in cyclohexane. The xerogel of **1** exhibits an emission band at approximately 610 nm (λ_{exc} = 470 nm). The xerogel of **1** also emits fluorescence in the red region of the spectrum in its films in the solid state. This data is significant taking into account that most luminophoric materials are used as solid films due to their practical applications. Therefore, the xerogel of **1** could be used to build fluorescent sensors.

In order to better understand the fluorescent properties and interactions, various experiments on dependence on the concentration of ¹H in RMN were conducted, using cyclohexane-d₁₂ as a deuterated solvent, demonstrating that the formation of organogellants occurs by means of very weak π-π type interactions, since a slight shift of the aromatic signals was observed.

X-ray diffraction (XRD) has great potential for elucidating the molecular structure of the organogels and can provide information on molecule assembly in the gel phase. XRD of the cyclohexane xerogel (2.75% by weight) has four reflections, three of which in the low angle region in 18.01, 9.00 and 6.00, with a laminar packing ratio corresponding to planes (001), (002) and (003).

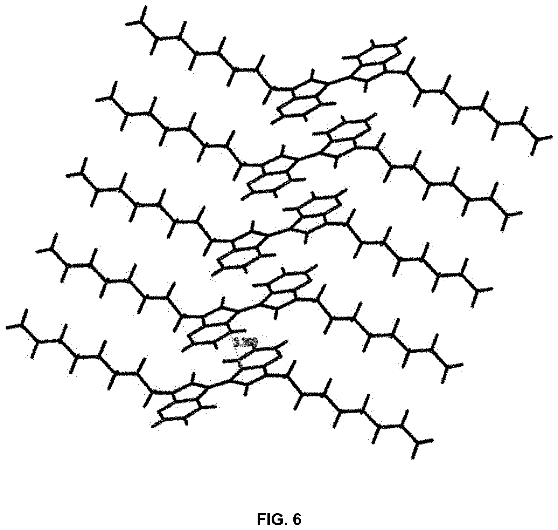

Furthermore, the crystalline structure of **1** was dissolved in acetone/dichloromethane (Table 1, Table 2, Figures 4, 5 and 6). Reddish crystals of **1** were obtained, adequate for single-crystal X-ray analysis, from slow evaporation in acetone/dichloromethane. The X-ray analysis indicates that species **1** crystallises in the monoclinic spatial group *P*2₁/*c*

**Table 1. Crystal 1 data**

| | |
|---|---|
| **Chemical formula** | C₃₀H₄₀N₄O₂ |
| **Molecular weight** | 488.66 |
| **Temperature** | 296(2) K |
| **Wavelength** | 0.71073 Å |
| **Crystal size** | 0.04 x 0.18 x 0.24 mm |
| **Crystal habit** | Light red plate |
| **Crystalline system** | monoclinic |
| **Spatial group** | *P*2₁/*c* |
| **a = 18.7376(11) Å** | α =90° |
| **b = 4.8671(3) Å** | β **=** 106.762(2)° |
| **Unit cell dimensions** | *a* **=** 18.7376(11) Å α **=** 90° |
| | *b* = 4.8671(3) Å β = 106.762(2)° |
| | *c* = 15.8639(8) Å γ = 90° |
| **Volume** | 1385.28(14) Å³ |
| **Z** | 2 |
| **Density (calculated)** | 1.172 Mg/cm³ |
| **Absorption coefficient** | 0.074 mm⁻¹ |
| **F(000)** | 528 |

**Table 2. Refining data of structure 1**

| | |
|---|---|
| **Theta range for the data collected** | 2.27 to 25.35° |
| **Ranges of hkl indices** | -22<=h<=17, -5<=k<=5, - 18<=l<=18 |
| **Reflections collected** | 14003 |
| **Independent reflections** | 2493 [R(int) = 0.0506] |
| **Coverage of independent reflections** | 98.7%. |
| **Absorption correction** | multi-scanning |
| **Max. and min. transmission coefficient** | 0.9970 and 0.9824 |
| **Structure solution technique** | *Direct methods* |
| **Structure solution program** | SHELXS-97 (Sheldrick, 2008) |
| **Refinement method** | full-matrix least square on F² method |
| **Refinement program** | SHELXL-97 (Sheldrick, 2008) |
| **Minimised function** | Σ w(Fo² - Fc²)² |
| **Data / Restrictions / Parameters** | 2493 / 0 / 165 |
| **Goodness-of-fit on F²** | 1.056 |
| **Δ/σ max** | 0.001 |
| **Final R indices** | 1569 data; I>2σ(I) R1 = 0.0564, wR2 = 0.1270 All data R1 = 0.1045, wR2 = 0.1618 |
| **Weighting scheme** | w=1/[σ²(Fₒ²)+(0.0925P)²+0.0000P ] where P=(Fₒ²+2F_{c}²)/3 |
| | |
| **Positive and negative peaks of greatest magnitude** | 0.392 and -0.337 eÅ⁻³ |
| **Greatest R.M.S deviation** | 0.147 eÅ⁻³ |

The monocrystalline structure of **1** revealed the planarity of the core and a significant intermolecular interaction between adjacent platforms. The crystals of compound **1** formed crystals in the form of very thin fibres. Compound **1** crystallises in the monoclinic spatial group *P*2₁/*c*. Only one half of the molecule in the asymmetrical unit, with an inversion centre disposed in the centre. The central ring is completely flat; coupling of the molecules in the crystal is achieved by means of π-π interactions that give rise to stair columns parallel to the b direction, where the adjacent molecules at a distance of 3.335 Å, with a slip angle of 43.28°. The slip angle is calculated as the angle between the long axis of a molecule and the centre line of adjacent molecules in the column. The packing of the columns in the crystal showed that the neighbour columns are inclined in the same direction as the angle, while molecule cores in adjacent columns in the c direction show a nearly perpendicular angle of 86.52°.

## Claims

1. A compound of formula (I): 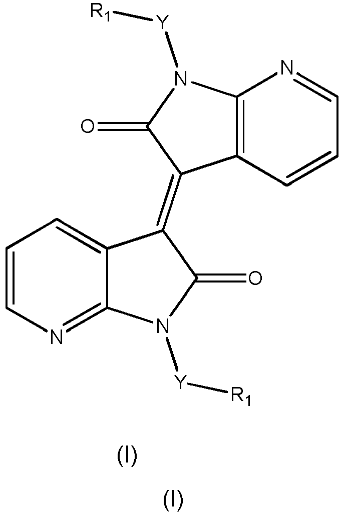 where Y is selected from CH₂, O, NH, C(O), S, S(O), NHC(O), (O)CNH and R₁ is a C1-C12 alkyl.

2. The compound, according to claim 1, where Y is CH₂.

3. The compound, according to the preceding claim, where R₁ is a C₃-C₁₁ alkyl.

4. The compound, according to the preceding claim, where R₁ is a heptyl.

5. The compound, according to any of the preceding claims, having the following formula:

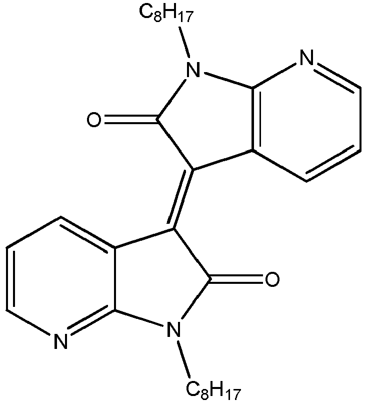

6. Use of a compound of formula (I) for manufacturing fluorescent materials.

7. An organogel, comprising at least one compound of formula (I) according to any of claims 1 to 5, **characterised in that** it emits fluorescence at a wavelength between 600 and 800 nm.

8. A xerogel, **characterised in that** it is the dessicated organogel according to claim 7.

9. A device comprising the xerogel according to claim 8.

10. Use of the xerogel, according to claim 8, for manufacturing optoelectronic devices.

11. Use of the xerogel, according to claim 8, for manufacturing fluorescent sensors.
